# EUROPEAN PATENT SPECIFICATION

(11) **EP 3 585 362 B1**
(45) Date of publication and mention of the grant of the patent: **01.04.2026**
(21) Application number: 18712651.1
(22) Date of filing: 26.02.2018
(51) Int. Cl.: A61K 9/50, A61K 31/14, A61K 31/198

(54) **COMPOSITION FOR CONTROLLED RELEASE OF PHYSIOLOGICALLY ACTIVE SUBSTANCES AND PROCESS FOR ITS PREPARATION**
ZUBEREITUNG ZUR KONTROLLIERTEN FREISETZUNG VON PHYSIOLOGISCH AKTIVEN STOFFEN UND DEREN HERSTELLUNGSVERFAHREN
COMPOSITION A LIBERATION CONTROLEE DE LA SUBSTANCE PHYSIOLOGIQUEMENT ACTIVE ET PROCEDE DE PREPARATION

(30) Priority: 27.02.2017 IT 201700021852
(43) Date of publication of application: 01.01.2020
(73) Proprietor: BALCHEM ITALIA S.r.l., 28040 Marano Ticino (NO) (IT)
(72) Inventor: GASPARI, Enrico, 47014 Meldola (FC) (IT); FARNEDI, Flavio, 47522 Cesena (FC) (IT); UNGHERI, Devis, 47121 Forlì (FC) (IT); VALENTINI, Arnaldo, 47521 Cesena (FC) (IT)
(74) Representative: Abthorpe, Mark
(86) International application number: PCT/IB2018/051189
(87) International publication number: WO 2018/154531

(56) References cited:
- WO-A1-2011/127236
- WO-A1-2011/140106
- WO-A2-2006/032958
- WO-A2-2008/015203
- WO-A2-2010/122583
- JP-A- S63 317 050
- US-A- 5 227 166
- US-A- 5 429 832

## Description

The present invention relates to a controlled release composition of physiologically active substances for zootechnical use. In particular, the subject of the present invention is a composition comprising microgranules capable of releasing in a controlled way the physiologically active substances they contain, a process for the production of such microgranules and their use in zootechnics.

It is known that physiologically active substances can be used to supplement or add additives to the diet of farm animals, in order to improve their health conditions, increase their productive longevity and increase their zootechnical performance.

The physiologically active substances thus used include for example amino acids, vitamins, enzymes, nutrients such as proteins and carbohydrates, probiotic microorganisms, prebiotic foods, mineral salts, choline and its derivatives.

Some of the above-mentioned physiologically active substances are already normally present in feedstuff used for animal feeding. However, sometimes the intake of these active substances, present in the diet, may be insufficient or inadequate to deal with deficiencies or situations of high productivity.

These physiologically active substances, having nutritive properties, are administered by mouth to the animals through formulated products (premixtures or complementary feedstuff) in which said active substances are "diluted" by mixing with a medium; the resulting product is then added to the feedstuff.

However, the active substances and feedstuff containing these active substances undergo an enzymatic chemical degradation in the first section of the animal digestive tract. In the case of ruminants (in which, before reaching the intestine, the food must pass through three forestomachs plus the glandular stomach) the degradation can be particularly intense owing to two main concomitant factors: a long transit time of the feedstuff in the forestomaches (especially in the rumen) and the presence of the microbial flora that performs a degradating action on most of the molecules passing through the rumen.

The action of ruminal microorganisms chemically alters some active substances, such as choline, transforming them into substances with a lower nutritional value or with a significantly reduced biological activity compared to the initial compound.

Moreover, the said active substances undergo further degradation during the preparation of the formulations, especially during the mixing, packaging and storage phases, as well as during the compaction treatments, due to the application of heat and/or pressure and/or steam.

In an attempt to provide solutions to release physiologically active substances in a rumen-protected form, i.e. protected from degradation in the ruminal environment, allowing the release of substances at a time following the rumen, several products have been proposed.

US3959493 describes a rumen by-pass product for oral administration to ruminants. Said product is in form of particles comprising an amount of a biologically active substance lower than 40% and a protective substance resistant to the ruminal environment and present in an amount of at least 60% by weight with respect to the weight of the particle. This protective substance is digestible in the ruminant lower digestive tract and consists of saturated and unsaturated aliphatic fatty acids, comprising from 14 to 22 carbon atoms.

US6797291 describes a method for stabilizing a hygroscopic ingredient in a wet composition, comprising the encapsulation of said hygroscopic ingredient in a lipid coating consisting for example of waxes, fatty acids, alcohols or esters derived from fatty acids, sterols, phospholipids and hydrogenated oils.

However, the products described in these documents are characterized by poor preservation stability, low concentrations of active substances and reduced post-ruminal digestibility.

US2011/250286 describes compositions in the form of microparticles or granules for the controlled release of physiologically active substances arranged in cores which are coated by a layer of fatty acids and a second layer of fatty acids and glycerides.

EP2274990 describes a method for producing a complementary ruminant feedstuff composition comprising the preparation of a mixture of a protective agent selected from animal or vegetal oils having a melting point of between 50°C and 90°C; lecithin and an amino acid; and the dripping of this mixture into water in order to make it solid.

US4876097 describes a composition comprising a coating with controlled hydrophilia and sensitive to pH variations comprising chemically modified cellulose.

US8906407 describes a composition comprising an ethyl-cellulose based coating.

However, the herein described compositions are poorly stable during the production of the feedstuff or the supplements where they are mixed with other ingredients which can remove their coating layer, thus making the product ineffective.

Another drawback of such known compositions is that mixing with an acidic ingredient, e.g. containing corn silage, may cause the coating to react even before the composition is administered to the animals.

EP1791532 describes a microgranule composition suitable for the controlled release of physiologically active substances in the ruminant post-ruminal tract, comprising a core and two coating layers. The first coating layer comprises a first hydrophobic substance selected from fats, fatty acids, hydrogenated oils, mono- and diglycerides of fatty acids, esters of fatty acids, fatty alcohols, with chains of 12 to 22 carbon atoms and a melting point from 40°C to 74°C. The second coating layer comprises a second hydrophobic substance selected from microcrystalline waxes, paraffin waxes, vegetal and synthetic waxes with a melting point of between 80°C and 100°C.

WO 2011/127236 discloses controlled release microparticles made of a core having choline chloride, L-Lysine and stearic acid and a layer made of hydrogenated vegetable oil and soy lecithin.

JP S63 317050 A aims to provide ruminant feed additives in which the biologically active substance, the main component of the nucleus, is protected from fermentation and degradation by microorganisms present in the rumen of ruminants, and in the digestive tract after the abomasum.

US 5 429 832 A describes: a coating composition containing the following (a), (b) and (c):
(a) at least one substance selected from the group consisting of linear or branched saturated fatty acids having from 14 to 22 carbon atoms, hardened animal oils and fats, hardened vegetable oils and fats, and waxes,
(b) chitosan, and
(c) at least one substance selected from the group consisting of unsaturated fatty acids and emulsifiers.

The composition described in this document guarantees a high degree of ruminal crossing (bypass), a good in vivo efficacy, excellent chemical, thermal and mechanical resistance, and high mixability and pelletability properties.

However, it is desirable to further improve the post-ruminal digestibility of the substances included in a microgranule composition, enhancing the total bioavailability of these substances.

The object of the present invention is thus to provide a composition for zootechnical use capable of releasing the substances contained therein in a controlled way, in the animal assimilation tracts namely from the abomasum to the small intestine, which does not have the drawbacks of the compositions according to the state of the art.

Said object is achieved with a rumen-resistant composition, the main features of which are specified in the first claim, a process for the preparation of such composition, the characteristics of which are specified in the related process claim, a feedstuff premixture comprising said composition, the characteristics of which are specified in the claim regarding a premixture, and a feedstuff composition comprising said premixture, the characteristics of which are specified in the respective claim. Other features according to the present invention are specified in the remaining dependent claims.

An advantage of the composition according to the present invention consists in the fact that it releases the physiologically active substances in the post-ruminal zone in a controlled way, achieving an improved total bioavailability of these substances.

In the context of the present invention, the term "ruminal by-pass" indicates the percentage amount by weight of substance reaching the animal post-ruminal area, namely the stomach and the intestine, with respect to the total amount by weight of the orally administered substance.

Whereas, the term "post-ruminal digestibility" of a substance means the percentage amount by weight of substance that is freed from the coating exclusively in the post-ruminal tract, dissolving and so making it digestible or absorbable, with respect to the total amount by weight of the orally administered substance.

The term "total bioavailability" of a substance is used to indicate the percentage amount by weight of substance that reaches the animal systemic circulation in a biologically active form, with respect to the total amount by weight of the orally administered substance.

Thus, a total bioavailability of the substance is defined by the combination of the two values of ruminal bypass and post-ruminal digestibility.

In fact, using methodologies known in the pharmaceutical industry, it has been established that the physiologically active substances administered by means of the compositions according to the present invention are bioavailable, in a non-degraded form, in percentages higher than those of the compositions according to the state of the art.

Furthermore, the composition for zootechnical use according to the present invention has controlled chemical and physical properties and is resistant to mechanical-structural degradation.

These and other advantages of the rumen-resistant composition and its production process according to the present invention will be evident to those skilled in the art from the following detailed description of some embodiments.

The rumen-resistant composition according to the present invention is in form of microgranules, wherein each microgranule comprises a core and one or more coating layers.

Said core contains one or more physiologically active ingredients or substances, generally in solid form, and a matrix.

Any substance that is to be administered to the animals in non-degraded form can in principle be contained in the core of the microgranules of the composition according to the present invention which physiologically active substance is selected from: methionine, choline, choline chloride, lysine, lysine hydro-chloride, lysine sulfate, vitamin C and its derivatives, vitamin A and its derivatives, vitamin E and its derivatives, vitamin D3 and its derivatives, vitamin B1 and its derivatives, vitamin B2 and its derivatives, vitamin B6 and its derivatives, vitamin B12, vitamin H, vitamin PP and its derivatives and a mixture of acids comprising lactic, fumaric, sorbic, formic, citric, malic, acetic, butyric and propionic acid.

These physiologically active substances can be used alone or mixed together in different weight ratios.

Said matrix, mixed with the physiologically active substance to obtain a uniform heterogeneous mixture, comprises at least one substance selected from substances having a binding action and inert substances.

The substances with binding action, used in the matrix, are non-toxic substances of vegetal or synthetic origin such as rubber, cellulose and its derivatives, amides and derivatives, waxes and derivatives and fats and derivatives.

Advantageously, vegetal waxes such as carnauba wax, rice wax, microcrystalline waxes, fats and their derivatives are used. In a preferred embodiment, the binder is a combination of rice wax and microcrystalline wax in a weight ratio of 1: 3 to 3: 1; advantageously 1: 1.

The inert ingredients, used in the formation of the matrix, are sliding substances and generally belong to the category of silicates, in particular hydrophobic silicates such as colloidal silica, synthetic amorphous silica, precipitated silica, sodium aluminum silicates, calcium silicate, talc, kaolin, hydrophobic synthetic zeolites.

The total amount by weight of binding substances and inert substances in the core of the composition according to the present invention is preferably between 1% and 70% of the total weight of the core. More preferably, the total amount of substances having a binding and inert action is between 10% and 30% by weight, and even more preferably it is between 12% and 17% by weight of the total core weight.

According to the invention, the core comprises at least one disintegrant agent. According to the invention the disintegrant agent is present in addition to a binding agent. In other words, a substance used as a disintegrant is added to the core in an amount such as to add a disruptive function.

This disintegrant agent is present in the core of the composition according to the present invention in an amount by weight preferably comprised between 1.5% and 6.5% of the total weight of the core. More preferably, the disgregant (agent) amount is in the range of 1.75% -5.75% by weight of the total weight of the core, and even more preferably, the amount by weight of disintegrant substances is comprised between 2% and 5% of the total weight of the core.

In the context of the present invention, the term disintegrant agent indicates a substance capable of modifying its own state or action in presence of a post-ruminal aqueous environment and causing a rapid disintegration of the core once it has interacted with a aqueous medium. These actions are, for example, an improved solubilization of the core substances, and/or an effervescent action that aids disruption or breakage of the core. In other words, the active ingredients may thus be provided with an improved post-ruminal digestibility thanks to the action of the disintegrant agents. The disruptive function can be carried out by a substance able to recall water in the post-ruminal environment and causing core disintegration.

In fact, it has surprisingly been found that said disintegrant agents improve the release of physiologically active substances once the composition comes into contact with the post-ruminal environment.

Disintegrant substances suitable for this purpose are vegetal lecithins selected from soy lecithin and sunflower lecithin.

Even more preferably, disintegrant agents include only soy lecithin which, as an emulsifier, has the function of recalling water towards the inside of the core and enhances the contact of the active ingredients with water, favouring the solubilization thereof.

Preferably, disintegrant agents include only sunflower lecithin.

The microgranules of the composition according to the present invention comprise at least one core coating layer. Preferably they comprise at least two coating layers. The external coating thus comprises at least a first layer composed of a substance forming a water-repellent film which is stable at the rumen environmental conditions for at least 8 hours and at least a second protective layer for protection against mechanical abrasion and impact, resistant to temperatures of between 75°C and 90°C.

According to an embodiment of the present invention, microgranules of the composition can be made in which said first and second layer are repeatedly deposited so as to form a covering comprising three, four or more coating layers.

Moreover, the microgranules of the composition according to the present invention can comprise further coating layers, having a composition different from that indicated for said first and second layer.

In fact, it has been found that this repeated and alternate arrangement of the first and second layer, and/or the application of further layers with different formulations, allows the water permeability of the microgranule to be modulated so that it remains waterproof for a period of time necessary for it to pass beyond the ruminal tract. This period of time may vary according to the size of the microgranules, their specific weight and buoyancy, i.e. their placement within the layers in which the rumen content is subdivided, but is normally within the range of 7-8 hours. Instead, the time which the microgranules spend within the area of post-ruminal digestive tracts, can be generally quantified in a further 24 hours.

The microgranules of the composition according to the present invention are advantageously impermeable for the first 10 to 12 hours of immersion in the digestive environment of ruminants, and subsequently they become more and more permeable to the external environment.

Said first coating layer of the core consists of one or more physiologically acceptable substances having hydrophobic properties.

The hydrophobic substances forming said first layer are selected from the group consisting of: fats, fatty acids, hydrogenated oils, mono and diglycerides of fatty acids, salts of fatty acids such as zinc, magnesium or calcium stearate, esters of fatty acids and fatty alcohols and their mixtures.

These hydrophobic substances are preferably selected from: lauric acid, stearic acid, palmitic acid, stearin, hydrogenated soybean oil, hydrogenated rapeseed oil, hydrogenated cotton oil, hydrogenated palm oil, hydrogenated linseed oil, sodium glyceryl mono-, di-, tri-stearate, calcium stearate, magnesium stearate, zinc stearate, stearyl alcohol, cetylstearyl alcohol.

Advantageously, the hydrophobic substances are selected from hydrogenated palm oil and/or hydrogenated rapeseed oil.

A second layer, arranged on the outside of said first layer, comprises at least one second physiologically acceptable substance, or a mixture of substances, having hydrophobic properties. Said second hydrophobic substance or mixture of substances is resistant to mechanical stress and maintains its chemical-physical properties unchanged up to a temperature of 80°C.

Said second hydrophobic substance included in said second layer is selected from the group consisting of microcrystalline waxes, paraffin waxes, vegetal waxes and edible synthetic waxes. Advantageously, said second hydrophobic substance is selected from the group consisting of rice wax, carnauba wax and/or microcrystalline waxes.

Advantageously, according to an embodiment of the present invention, at least one coating layer further comprises a polluting substance, that is a substance altering the characteristics that said coating layer would have in its absence. Preferably, the pollutant is selected from the group consisting of emulsifying substances such as vegetal lecithins, ethoxylated oils and alginates, and/or in the group of fatty acids and/or in the group of methacrylate copolymers. As emulsifying substances in the coating layer or layers, soy or sunflower lecithin, ethoxylated castor oil, alkali metal and magnesium alginates can advantageously be used. Preferably the emulsifying substance is between 0.1% and 6% by weight of the coating layer weight.

As fatty acids polluting in the coating layer or layers, palmitic acid, oleic acid, linoleic acid, linolenic acid, stearic acid, or a combination thereof can advantageously be used. Preferably the inclusion of fatty acids or a combination thereof is between 1% and 40% by weight of the coating layer weight. In particular they are single fatty acids, which are much more digestible than hydrogenated waxes and triglycerides that are used as coating layers, so they tend to be more easily digested and, consequently, over time, to create micro-canals (lesions) in the structure of the coating, allowing water to enter gradually.

Polymethyl methacrylate (PMMA) can advantageously be used as methacrylate copolymers polluting the coating layer or layers.

Preferably, emulsifying substances include soy or sunflower lecithin. These substances have the function of letting water slowly enter the coating layers.

In one embodiment, emulsifying substances include ethoxylated castor oil.

In one embodiment, emulsifying substances include sodium or magnesium alginate. This substance is more digestible than waxes and triglycerides, and tends to be more easily digested and, consequently, over time, to create micro-channels (lesions) in the structure of the coating allowing water to enter gradually.

In one embodiment, polluting fatty acids include palmitic acid, which tends to be easily digested and, as a result, over time, tends to create micro-channels (lesions) in the coating structure, allowing water to enter gradually.

In one embodiment, polluting fatty acids include oleic acid.

In one embodiment, polluting fatty acids include linoleic acid.

In one embodiment, polluting fatty acids include linolenic acid.

In one embodiment, polluting fatty acids include stearic acid.

In one embodiment, polluting fatty acids include a combination of the aforementioned fatty acids.

In one embodiment, pollutants include polymethyl methacrylate (PMMA): pH-dependent polymer which is solubilised at acidic (stomach) pH, while it remains solid between weakly acid pH (5) and weakly basic pH (8): this range comprising the pH values found in the rumen of healthy animals. As a consequence, if it is wetted by a stomach solution it dissolves, but it does not dissolve if wetted by a rumen solution.

In a preferred form or in preferred forms of the present disclosure, the rumen-resistant composition according to the present disclosure includes any combination of disintegrant agents in the core, as defined above and pollutants, as defined above in the coating layer.

It follows that this combination allows an improved post-ruminal digestibility to be obtained compared to the products without it; said improved post-ruminal digestibility being conferred to the active ingredients owing to the synergistic action between disintegrant agents in the core and polluting agents in the coating layers.

According to a preferred embodiment of the invention, the microgranules comprise at least two coating layers, in which each coating layer is as in any of the above defined embodiments or combinations thereof. Each coating layer comprises a quantity of pollutant comprised between 0.01% and 40% by weight of the weight of the coating layer.

In each microgranule the overall weight of the coating layers is optionally between 10% and 50% of the weight of the microgranule, more preferably the overall weight of the coating layers is between 15% and 40% of the weight of the microgranule.

The present invention also relates to a process for the preparation of the microgranule composition described above.

The process comprises a phase of mixing the substances forming the core, i.e. the physiologically active substances, the matrix substances and disintegrant substances, which is carried out in a container equipped with mixing, cutting, heating and cooling. Mixing is carried out until a homogeneous mixture is obtained.

In case of a physiologically active substance with a high microgranulometry, a preliminary micronization treatment may be necessary. As highlighted above, the core may comprise one or more physiologically active substances and the resulting mixture must be solid enough for the cores to remain intact during the subsequent processing steps, particularly during the coating operations.

The process for preparing the composition of microgranules according to the present invention comprises a subsequent step of extrusion of the mixture thus obtained.

The mixture obtained is extruded into an extruder so as to obtain microgranules of the required shape and size.

The extruder, known to those skilled in the art, is advantageously a bi-screw extruder provided with feeding and mixing, and sectors provided with heating and cooling means that allow application of a temperature gradient based on the treatment carried out. The head of the extruder is equipped with a heatable and/or coolable die and provided with a plurality of outlet holes with a predetermined diameter.

Outside the die the composition is subjected to cutting with a cutter with variable speed that allows the length of the microgranule core to be adjusted.

The core thus obtained is subjected to cooling and sieving to remove powders and waste matter of undesirable dimensions.

At this point the cores can be subjected to the coating step.

In an alternative embodiment of the present invention, before the coating step, the core obtained after cooling and sieving is subjected to a spheronization operation or to another treatment for modifying its shape. The spheronization operation can be carried out with a rotating disk spheronizer, blowing in air and adjuvants such as softeners, thickeners and aggregating agents.

The spheroidal shape has the advantage of greater compactness, smoothness, coatibility and mixability, compared to the cylindrical shape.

Indeed, according to the present invention, it has been found that the spheroidal shape allows a better uniformity of the coating to be obtained, this allowing a more effective protective action of the coating for the same thickness of the applied protective layers.

The cores of the rumen-resistant composition according to the present invention can alternatively be obtained through the following techniques:
- dry and/or wet composition, wherein the phsyological active substances are mixed with the matrix substances until forming irregular microgranules;
- compression together with the matrix substances;
- dripping: wherein the phsyological active substance are dispersed in waxes or melted waxes forming a mixture that is dripped in a cooling liquid;
- spray-cooling: wherein the phsyological active substance are dispersed in the melted matrix and sprayed in a cooling tower;
- coacervation: wherein the phsyological active substance are melted in a solution with the matrix and then injected in a solution of water and hardener (for example CaCl₂). The sphere thus obtained is then dried and potentially coated.

According to an embodiment of the invention, the core of the microgranules of the rumen-resistant composition has a cylindrical shape, the height of which is comprised between 0.5 mm and 2 mm. According to an alternative embodiment, the core of the microgranules of the rumen-resistant composition has a substantially spheroidal shape, the diameter of which is comprised between 0.5 mm and 2 mm.

Subsequently, the cores thus obtained are subjected to a coating step. The coating layers can be applied in a pan, by a coating technique known to those skilled in the art. Alternatively, fluid bed coating techniques can be used, for example top spray fluid bed, bottom spray fluid bed or tangential spray fluid bed; or spray techniques with a single component or a mixture of components, or the dry-coating technique.

Further advantages and characteristics according to the present invention will be clear to those skilled in the art from the following detailed and non-limiting description of an embodiment thereof with reference to the following examples.

### EXAMPLES

For the following examples, the following analysis and inspection tools were used:
- Sieves, arranged at the end of each single production step, for the microgranule dimension line check;
- Microscope for visual checks;
- Melting point gauge for thermal resistance check;
- Penetrometer for hardness and mechanical resistance check;
- Automatic titrator or HPLC for the quantitative determination of the active ingredients (concentration);
- Pharmaceutical dissolver for determining the degree of ruminal bypass. The experimental conditions of use were 39°C, 15 rpm, 8 hours presence in the solution with "ruminal" pH of about 6.8.
- Daisy^{II} ANKOM: commercially available laboratory artificial rumen, for determining the degree of ruminal bypass. The experimental conditions of use were 39°C, 8 hours presence in ruminal conditions, buffer solution at "ruminal" pH with insertion of "ruminal inoculum" and re-creation of ruminal anaerobiosis according to a university procedure known to those skilled in the art. The pH normally tested in the quality control phase is 6.8. Pharmaceutical dissolver for determining the degree of post-ruminal digestibility. The experimental conditions of use followed the procedure known to experts in the field as "Boisen Test" and, in particular, they were:
   - 39°C, 30 rpm, 2h, buffer solution with "gastric" pH (2.0) + pepsin inoculum.
   - 39°C, 30 rpm, 4h, buffer solution with "intestinal" pH (6.8) + pancreatine inoculum.
   - 39°C, 30 rpm, 18h, buffer solution with "intestinal" pH (6.8) + lipase and bile extract inoculum.

### Example 1 - Preparation of microgranules containing choline chloride

### Example 1.1

425 kg of choline chlorine with a purity of 99% were mixed with 10 kg of spray rice wax, 15 kg of zinc stearate, 10 kg of soy lecithin and 40 kg of silica. The mixture was extruded using an extruder with sectors at different temperature gradients according to the following program:

| Sector 1 | Sector 2 | Sector 3 | Sector 4 | **Sector** 5 | Sector 6 |
|---|---|---|---|---|---|
| 85°C | 85°C | 50°C | 50°C | 45°C | 65°C |

The cores thus obtained had a concentration of 85% in choline chloride. The cores were subsequently subjected to coating in a pan.

A first coating layer was formed, coating 400 kg of microgranules with 120 kg of a coating mixture comprising:
- 65% by weight of hydrogenated palm oil;
- 32% by weight of palmitic acid;
- 3% by weight of soy lecithin.

A second coating layer was then formed, coating the microgranules coated by the first layer with 40 kg of a coating mixture comprising:
- 50% by weight of hydrogenated palm oil;
- 50% by weight of rice wax.

All the above indicated percentages are percentages by weight based on the total weight of the covering layer.

The microgranules thus obtained had a concentration of 60% by weight of choline chloride with respect to the total weight of the microgranules.

Then the microgranules were subj ected to an evaluation of the degree of ruminal by-pass, post-ruminal digestibility and total bioavailability using the Boisen method.
For a complete description of the Boisen method, reference is made to the publication S. Boisen, J. A. Fernàndez "Prediction of total tract digestibility of energy feedstuffs and pig diets by in vitro analyses" Animal Feed Science Technology 68, 277-286, 1997*.*

The in vitro results are highlighted in Table 1.

**Table 1**

| Sample | Content (%) | Degree of ruminal by-pass (*) | Digestibility (**) | Bioavailability (**) |
|---|---|---|---|---|
| 1 | 59.4 | 80.3 | 89.3 | 71.7 |

### Example 1.2

425 kg of choline chlorine with a purity of 99% were mixed with 10 kg of spray rice wax, 15 kg of zinc stearate, 10 kg of soy lecithin, 5 kg of citric acid, 5kg of sodium bicarbonate and 30 kg of silica. The mixture was extruded using an extruder with sectors at different temperature gradients according to the following program:

| Sector 1 | Sector 2 | Sector 3 | Sector 4 | Sector 5 | Sector 6 |
|---|---|---|---|---|---|
| 70°C | 75°C | 80°C | 80°C | 80°C | 85°C |

The cores thus obtained had a concentration of 85% in choline chloride. The cores were subsequently subjected to coating in a pan.

A first coating layer was formed, coating 400 kg of microgranules with 100 kg of a coating mixture comprising:
- 75% by weight of hydrogenated palm oil;
- 23% by weight of palmitic acid;
- 2% by weight of ethoxylated castor oil.

A second coating layer was then formed, coating the microgranules coated by the first layer with 60 kg of a coating mixture comprising:
- 50% by weight of hydrogenated rapeseed oil;
- 45% by weight of carnauba wax;
- 5% by weight of polymethyl methacrylate.

All the above indicated percentages are percentages by weight based on the total weight of the covering layer.

The microgranules thus obtained had a concentration of 60% by weight of choline chloride with respect to the total weight of the microgranules.

Then the microgranules were subj ected to an evaluation of the degree of ruminal by-pass, post-ruminal digestibility and total bioavailability using the Boisen method. For a complete description of the Boisen method, reference is made to the publication S. Boisen, J. A. Fernàndez "Prediction of total tract digestibility of energy feedstuffs and pig diets by in vitro analyses" Animal Feed Science Technology 68, 277-286, 1997*.*

The in vitro results are highlighted in Table 2.

**Table 2**

| Sample | Content (%) | Degree of ruminal by-pass (*) | Digestibility (**) | Bioavailability (**) |
|---|---|---|---|---|
| 2 | 59.7 | 78.2 | 92 | 71.9 |

### Example 1.3

The extruded cores obtained with the procedure of Example 1.2 were spheronized with the use of an aqueous 75% choline chloride solution as an adjuvant for spheronization and subsequently they were coated in a pan.

A first coating layer was formed, coating 400 kg of spheroidal microgranules with 125 kg of a coating mixture comprising:
- 72% by weight of hydrogenated palm oil;
- 25% by weight of palmitic acid;
- 3% by weight of ethoxylated castor oil.

A second coating layer was then formed, coating the microgranules coated by the first layer with 20 kg of a coating mixture comprising:
- 50% by weight of hydrogenated palm oil;
- 50% by weight of rice wax.

Finally, a third coating layer of 20 kg containing 100% of polymethyl methacrylate was formed.

All the above indicated percentages are percentages by weight based on the total weight of the covering layer.

The microgranules thus obtained had a concentration of 60% by weight of choline chloride with respect to the total weight of the microgranules.

The in vitro results are highlighted in Table 3.

**Table 3**

| Sample | Content (%) | Degree of ruminal by-pass (*) | Digestibility (**) | Bioavailability (**) |
|---|---|---|---|---|
| 3 | 61.4 | 86.4 | 85.0 | 73.4 |

### Example 2 - Preparation of microgranules containing Lysine HCl

### Example 2.1

400 kg of micronized lysine hydrochloride were mixed with 60 kg of granulated rice wax, 48 kg of powdered powder, and 12 kg of soy lecithin. The mixture was extruded using an extruder with sectors at different temperature gradients according to the following program:

| Sector 1 | Sector 2 | Sector 3 | Sector 4 | Sector 5 | Sector 6 |
|---|---|---|---|---|---|
| 85°C | 65°C | 50°C | 50°C | 50°C | 50°C |

The cores thus obtained had a concentration of 75% in lysine hydrochloride. The cores were subsequently subjected to coating in a pan.

A first coating layer was formed, coating 350 kg of microgranules with 100 kg of a coating mixture comprising:
- 95% by weight of hydrogenated palm oil;
- 5% by weight of sunflower lecithin.

A second coating layer was then formed, coating the microgranules coated by the first layer with 75 kg of a coating mixture comprising:
- 50% by weight of hydrogenated rapeseed oil;
- 48% by weight of carnauba wax;
- 2% by weight of sunflower lecithin.

All the above indicated percentages are percentages by weight based on the total weight of the covering layer.

The microgranules thus obtained had a concentration of 50% by weight of lysine hydrochloride with respect to the total weight of the microgranules.

Then the microgranules were subj ected to an evaluation of the degree of ruminal by-pass, post-ruminal digestibility and total bioavailability using the Boisen method.
For a complete description of the Boisen method, reference is made to the publication S. Boisen, J. A. Fernàndez "Prediction of total tract digestibility of energy feedstuffs and pig diets by in vitro analyses" Animal Feed Science Technology 68, 277-286, 1997*.*

The in vitro results are highlighted in Table 4.

**Table 4**

| Sample | Content (%) | Degree of ruminal by-pass (*) | Digestibility (**) | Bioavailability (**) |
|---|---|---|---|---|
| 4 | 50.2 | 73.4 | 86 | 63.1 |

### Example 2.2

324 kg of micronized lysine hydrochloride were mixed with 64 kg of rice wax spray and 12 kg of soy lecithin. The mixture was extruded using an extruder with sectors at different temperature gradients according to the following program:

| Sector 1 | Sector 2 | Sector 3 | Sector 4 | Sector 5 | Sector 6 |
|---|---|---|---|---|---|
| 85°C | 85°C | 80°C | 80°C | 80°C | 80°C |

The cores thus obtained had a concentration of 80% lysine hydrochloride. The cores were spheronized, using a 50% lysine hydrochloride solution in water as a spheronization adjuvant, and subsequently subjected to coating in a pan.

A first coating layer was formed, coating 350 kg of microgranules with 90 kg of a coating mixture comprising:
- 75% by weight of hydrogenated palm oil;
- 23% by weight of palmitic acid;
- 2% by weight of ethoxylated castor oil.

A second coating layer was then formed, coating the microgranules coated by the first layer with 50 kg of a coating mixture comprising:
- 48% by weight of hydrogenated rapeseed oil;
- 52% by weight of rice wax;
- 2% by weight of sunflower lecithin.

A third coating layer was then formed, coating the microgranules coated by the second layer with 40 kg of a coating mixture identical to that of the first layer.

A fourth coating layer was then formed, coating the microgranules coated by the third layer with 30 kg of a coating mixture identical to that of the second layer.

All the above indicated percentages are percentages by weight based on the total weight of the covering layer.

The microgranules thus obtained had a concentration of 50% by weight of lysine hydrochloride with respect to the total weight of the microgranules.

Then the microgranules were subj ected to an evaluation of the degree of ruminal by-pass, post-ruminal digestibility and total bioavailability using the Boisen method. For a complete description of the Boisen method, reference is made to the publication S. Boisen, J. A. Fernàndez "Prediction of total tract digestibility of energy feedstuffs and pig diets by in vitro analyses" Animal Feed Science Technology 68, 277-286, 1997*.*

The in vitro results are highlighted in Table 5.

**Table 5**

| Sample | Content (%) | Degree of ruminal by-pass (*) | Digestibility (**) | Bioavailability (**) |
|---|---|---|---|---|
| 5 | 49.5 | 78 | 91 | 71 |

### Example 3 - Preparation of microgranules containing DL-Methionine

### Example 3.1

430 kg of DL-methionine were mixed with 30 kg of rice wax spray, 25 kg of hydrogenated palm oil, 5 kg of silica, 5 kg of citric acid and 5 kg of sodium bicarbonate. The mixture was extruded using an extruder with sectors at different temperature gradients according to the following program:

| Sector 1 | Sector 2 | Sector 3 | Sector 4 | Sector 5 | Sector 6 |
|---|---|---|---|---|---|
| 85°C | 45°C 40°C | 40°C 35°C | 60°C | | |

The cores thus obtained had a concentration of 85% DL-methionine. The cores were subsequently subjected to coating in a pan.

A first coating layer was formed, coating 400 kg of microgranules with 40 kg of a coating mixture comprising:
- 70% by weight of hydrogenated rapeseed oil;
- 30% by weight of linolenic acid.

A second coating layer was then formed, coating the microgranules coated by the first layer with 35 kg of a coating mixture comprising:
- 35% by weight of hydrogenated palm oil;
- 65% by weight of carnauba wax.

A third coating layer was then formed, coating the microgranules coated by the second layer with 10 kg of a coating mixture identical to that of the first layer.

All the above indicated percentages are percentages by weight based on the total weight of the covering layer.

The microgranules thus obtained had a concentration of 70% by weight of DL-Methionine with respect to the total weight of the microgranules.

Then the microgranules were subj ected to an evaluation of the degree of ruminal by-pass, post-ruminal digestibility and total bioavailability using the Boisen method. The in vitro results are highlighted in Table 6.

**Table 6**

| Sample | Content (%) | Degree of ruminal by-pass (*) | Digestibility (**) | Bioavailability (**) |
|---|---|---|---|---|
| 6 | 69.3 | 82.3 | 75.0 | 61.7 |

### Example 3.2

Further 400 kg of extruded granules obtained following the same procedure described in the first part of the previous example were coated in a pan using 3 coating layers.

A first coating layer was formed, coating 400 kg of microgranules with 35 kg of a coating mixture comprising:
- 82% by weight of hydrogenated palm oil;
- 18% by weight of linolenic acid.

A second coating layer was then formed, coating the microgranules coated by the first layer with 35 kg of a coating mixture comprising:
- 52% by weight of hydrogenated rapeseed oil;
- 45% by weight of carnauba wax;
- 3% by weight of sunflower lecithin.

A third coating layer was then formed, coating the microgranules coated by the second layer with 15 kg of a coating mixture comprising:
- 50% by weight of hydrogenated palm oil;
- 49% by weight of rice wax;
- 1% of ethoxylated castor oil.

All the above indicated percentages are percentages by weight based on the total weight of the covering layer.

The microgranules thus obtained had a concentration of 70% by weight of DL-Methionine with respect to the total weight of the microgranules.

Then the microgranules were subj ected to an evaluation of the degree of ruminal by-pass, post-ruminal digestibility and total bioavailability using the Boisen method. The in vitro results are highlighted in Table 7.

**Table 7**

| Sample | Content (%) | Degree of ruminal by-pass (*) | Digestibility (**) | Bioavailability (**) |
|---|---|---|---|---|
| 7 | 69.2 | 78.8 | 90 | 70.9 |

### Example 4 - Preparation of microgranules containing L-lysine, nicotinic acid and DL-Methionine

### Example 4.1

180 kg of 99% choline chloride were mixed with 240 kg of 99% L-lysine hydrochloride, 65 kg of nicotinic acid (vitamin PP) and 145 kg of D, L-methionine. 20 kg of citric acid, 20 kg of sodium bicarbonate, 5 kg of sunflower lecithin and 225 kg of rice wax spray were added. The mixture was extruded.

The cores thus obtained had a concentration of 20% of choline chloride, 26.7% of L-lysine hydrochloride, 7.2% of nicotinic acid, 16.1% of D, L-methionine.

The nuclei were subjected to coating in a pan.

A first coating layer was formed, coating 400 kg of microgranules with 200 kg of a coating mixture comprising:
- 66% by weight of hydrogenated rapeseed oil;
- 34% by weight of palmitic acid.

A second coating layer was then formed, coating the microgranules coated by the first layer with 100 kg of a coating mixture comprising:
- 48% by weight of hydrogenated palm oil;
- 50% by weight of rice wax;
- 2% by weight of soy lecithin.

### Example 5

Using the above mentioned analytical methods (pharmaceutical dissolver), in vitro tests were carried out to determine the degree of by-pass and the bioavailability of choline chloride:
- a product A consisting of choline chloride, 99% pure;
- a product B consisting of microencapsulated chlorine choline granules obtained by means of spray-cooling technology, containing 25% by weight of choline chloride with respect to the total weight of the granule;
- a product C consisting of microgranules obtained according to Example 4 of the patent EP1791532, containing 50% by weight of choline chloride with respect to the total weight of the granule;
- a product D consisting of microgranules obtained according to Example 1.3 described above.

Test results are shown in the following Tables 8 and 9.

Table 8 shows the results obtained considering a theoretical administration of 200g of product.

**Table 8**

| Product | Choline Chloride administered (g) | | Choline Chloride lost in the rumen in 8h (g) | | Bypassed Choline Chloride (g) | | **Bioavailable Choline Chloride (g)** | | Choline Chloride in faeces (g) | |
|---|---|---|---|---|---|---|---|---|---|---|
| A | | 198 | | 198 | | 0 | | **0** | | 0 |
| B | | 50 | | 37.5 | | 12.5 | | **12.5** | | 0 |
| C | | 100 | | 20 | | 80 | | **40** | | 40 |
| D | | 120 | | 17 | | 103 | | **88** | | 15 |

Table 8 9 shows the results obtained with a theoretical administration of 100g of choline chloride.

**Table 9**

| Product | Product to be administered (g) | | Choline Chloride lost in the rumen in 8h (g) | | Bypassed Choline Chloride (g) | | **Bioavailable Choline Chloride (g)** | | Choline Chloride in faeces (g) | |
|---|---|---|---|---|---|---|---|---|---|---|
| A | | 101 | | 101 | | 0 | | **0** | | 0 |
| B | | 400 | | 75 | | 25 | | **25** | | 0 |
| C | | 200 | | 20 | | 80 | | **40** | | 40 |
| D | | 167 | | 13.5 | | 86.5 | | **73** | | 13.5 |

### Example 6

Using the above mentioned analytical methods (pharmaceutical dissolver), in vitro tests were carried out to determine the degree of by-pass and the bioavailability of choline chloride:
- a product A consisting of choline chloride, 99% pure;
- a product B consisting of microencapsulated chlorine choline granules obtained by means of spray-cooling technology, containing 25% by weight of choline chloride with respect to the total weight of the granule
- a product C consisting of microgranules obtained according to Example 4 of the patent EP1791532, containing 50% by weight of choline chloride with respect to the total weight of the granule;
- a product D consisting of microgranules obtained according to Example 1.3 described above;
- a product E consisting of microgranules obtained according to the extruded form of Example 1.3, but coated with coating layers devoid of pollutants;
- a product F consisting of microgranules obtained according to the extruded form of Example 1.3, in which the portions of disintegrants were replaced by rice wax spray (binder), but the coating layers were "polluted" with twice the amount of pollutants compared to Example 1.3.

The results of the tests are shown in the following tables 10 and 11. The tables show that the mere presence of the disintegrant is effective in making the choline bioavailable in the post-ruminal phase.

Table 10 shows the results obtained considering a theoretical administration of 200g of product.

**Table 10**

| Product | Choline Chloride administered (g) | | Choline Chloride lost in the rumen in 8h (g) | | Bypassed Choline Chloride (g) | | **Bioavailable Choline Chloride (g)** | | Choline Chloride in faeces (g) | |
|---|---|---|---|---|---|---|---|---|---|---|
| A | | 198 | | 198 | | 0 | | **0** | | 0 |
| B | | 50 | | 37.5 | | 12.5 | | **12.5** | | 0 |
| C | | 100 | | 20 | | 80 | | **40** | | 40 |
| D | | 120 | | 17 | | 103 | | **88** | | 15 |
| E | | 120 | | 12 | | 108 | | **54** | | 54 |
| F | | 120 | | 45 | | 75 | | **55** | | 20 |

Table 11 shows the results obtained considering a theoretical administration of 100g of choline chloride.

**Table 11**

| Product | Product to be administered (g) | | Choline Chloride lost in the rumen in 8h (g) | | Bypassed Choline Chloride (g) | | **Bioavailable Choline Chloride (g)** | | Choline Chloride in faeces (g) | |
|---|---|---|---|---|---|---|---|---|---|---|
| A | | 101 | | 101 | | 0 | | **0** | | 0 |
| B | | 400 | | 75 | | 25 | | **25** | | 0 |
| C | | 200 | | 20 | | 80 | | **40** | | 40 |
| D | | 167 | | 13.5 | | 86.5 | | **73** | | 13.5 |
| E | | 167 | | 9.6 | | 90.4 | | **47** | | 43.4 |
| F | | 167 | | 36.9 | | 63.1 | | **45.3** | | 17.8 |

## Claims

1. A microgranule comprising:
i) a core comprising:
a) one or more physiologically active substances selected from the group consisting of methionine, choline, choline chloride, lysine, lysine hydro-chloride, lysine sulfate, vitamin C , vitamin A, vitamin E , vitamin D3 , vitamin B1 , vitamin B2 , vitamin B6 , vitamin B12, vitamin H, vitamin PP and a mixture of acids comprising lactic, fumaric, sorbic, formic, citric, malic, acetic, butyric and propionic acid; and
b) a matrix comprising at least one of a substance having a binding action and an inert substance, wherein:
the substance having a binding action is of vegetal or synthetic origin and is rubber, cellulose, an amide, a wax, or a fat; and
the inert substance is a silicate, colloidal silica, synthetic amorphous silica, precipitated silica, a sodium alumina silicate, calcium silicate, talc, kaolin, or a hydrophobic synthetic zeolite; and
c) at least one disintegrant agent that is a vegetal lecithin selected from soy lecithin and sunflower lecithin;
wherein the at least one disintegrant agent is present in amount by weight of between 1.5% and 6.5% with respect to the total weight of the core
ii) at least one first core coating layer comprising one or more hydrophobic substances selected from the group consisting of: fats, fatty acids, hydrogenated oils, mono and diglycerides of fatty acids, salts of fatty acids (such as zinc, magnesium or calcium stearate), esters of fatty acids and fatty alcohols and their mixtures.

2. The microgranule according to claim 1, wherein the total amount of the substance having a binding action and the inert substance is between 10% and 30% by weight of the total core weight.

3. The microgranule according to any one of the previous claims, wherein the at least one disintegrant agent is soy lecithin.

4. The microgranule according to claim 1 or claim 2, wherein the at least one disintegrant agent is sunflower lecithin.

5. The microgranule according to any one of the previous claims, wherein said core has a cylindrical shape, the height of which is comprised between 0.5 mm and 2 mm or a spheroidal shape, the diameter of which is comprised between 0.5 mm and 2 mm.

6. The microgranule according to any one of the previous claims wherein the total weight of the coating layers is between 15% and 40% of the microgranule weight.

7. The microgranule according to any one of the previous claims, wherein the at least one first core coating layer comprises one or more hydrophobic substances selected from: lauric acid, stearic acid, palmitic acid, stearin, hydrogenated soybean oil, hydrogenated rapeseed oil, hydrogenated cotton oil, hydrogenated palm oil, hydrogenated linseed oil, sodium glyceryl mono-, di-, tri-stearate, calcium stearate, magnesium stearate, zinc stearate, stearyl alcohol and cetylstearyl alcohol.

8. The microgranule according to any one of the previous claims wherein the at least one first core coating layer comprises at least one polluting substance selected from the group of emulsifying substances consisting of vegetal lecithin, ethoxylated oils or alginates and/or from the group consisting of fatty acids and/or from the group formed by methacrylate polymers.

9. The microgranule according to claim 8, wherein said emulsifying substance is selected from the group consisting of soy or sunflower lecithin, ethoxylated castor oil, alkali metal and magnesium alginates.

10. The microgranule according to claim 8, wherein said polluting fatty acid is selected from the group consisting of palmitic acid, oleic acid, linoleic acid, linolenic acid, stearic acid or a combination thereof.

11. A process for the preparation of a microgranule according to any one of the previous claims, comprising the steps of:
- extruding a mixture comprising one or more physiologically active substances selected from the group consisting of methionine, choline, choline chloride, lysine, lysine hydro-chloride, lysine sulfate, vitamin C , vitamin A , vitamin E , vitamin D3 , vitamin B1 , vitamin B2 , vitamin B6 , vitamin B12, vitamin H, vitamin PP and a mixture of acids comprising lactic, fumaric, sorbic, formic, citric, malic, acetic, butyric and propionic acid; binding substances, inert substances and extrusion adjuvants, and disintegrant agents;
- optionally subjecting the microgranule to spheronization;
- forming one or more coating layers.

12. A premixture for animal feedstuff comprising a microgranule according to any one of claims 1 to 10.

13. A feedstuff comprising the premixture according to claim 12.

## Patentansprüche

1. Mikrogranulat, das Folgendes umfasst:
i) einen Kern, der Folgendes umfasst:
a) eine oder mehrere physiologisch aktive Substanzen, ausgewählt aus der aus Methionin, Cholin, Cholinchlorid, Lysin, Lysinhydrochlorid, Lysinsulfat, Vitamin C, Vitamin A, Vitamin E, Vitamin D3, Vitamin B1, Vitamin B2, Vitamin B6, Vitamin B12, Vitamin H, Vitamin PP und einer Milchsäure, Fumarsäure, Sorbinsäure, Ameisensäure, Citronensäure, Äpfelsäure, Essigsäure, Buttersäure und Propionsäure umfassenden Mischung von Säuren bestehenden Gruppe, und
b) eine Matrix, die eine Substanz mit einer bindenden Wirkung und/oder eine inerte Substanz umfasst, wobei:
die Substanz mit bindender Wirkung pflanzlichen oder synthetischen Ursprungs ist und Gummi, Cellulose, ein Amid, ein Wachs oder ein Fett ist und
die inerte Substanz ein Silikat, kolloidales Siliciumdioxid, synthetisches amorphes Siliciumdioxid, gefälltes Siliciumdioxid, ein Natriumaluminiumsilikat, Calciumsilikat, Talk, Kaolin oder ein hydrophober synthetischer Zeolith ist, und
c) mindestens ein Sprengmittel, bei dem es sich um ein pflanzliches Lecithin, ausgewählt aus Sojalecithin und Sonnenblumenlecithin, handelt,
wobei das mindestens eine Sprengmittel in einer Menge von 1,5 Gew.-% bis 6,5 Gew.-%, bezogen auf das Gesamtgewicht des Kerns, vorliegt,
ii) mindestens eine erste Kernüberzugsschicht, die eine oder mehrere hydrophobe Substanzen, ausgewählt aus der aus Fetten, Fettsäuren, hydrierten Ölen, Mono- und Diglyceriden von Fettsäuren, Salzen von Fettsäuren (wie Zink-, Magnesium- oder Calciumstearat), Estern von Fettsäuren und Fettalkoholen und deren Mischungen bestehenden Gruppe, umfasst.

2. Mikrogranulat nach Anspruch 1, wobei die Gesamtmenge der Substanz mit einer bindenden Wirkung und der inerten Substanz 10 Gew.-% bis 30 Gew.-% des Gesamtkerngewichts beträgt.

3. Mikrogranulat nach einem der vorhergehenden Ansprüche, wobei das mindestens eine Sprengmittel Sojalecithin ist.

4. Mikrogranulat nach Anspruch 1 oder Anspruch 2, wobei das mindestens eine Sprengmittel Sonnenblumenlecithin ist.

5. Mikrogranulat nach einem der vorhergehenden Ansprüche, wobei der Kern eine zylindrische Form, deren Höhe 0,5 mm bis 2 mm beträgt, oder eine kugelförmige Form, deren Durchmesser 0,5 mm bis 2 mm beträgt, aufweist.

6. Mikrogranulat nach einem der vorhergehenden Ansprüche, wobei das Gesamtgewicht der Überzugsschichten 15 % bis 40 % des Mikrogranulatgewichts beträgt.

7. Mikrogranulat nach einem der vorhergehenden Ansprüche, wobei die mindestens eine erste Kernüberzugsschicht eine oder mehrere aus Laurinsäure, Stearinsäure, Palmitinsäure, Stearin, hydriertem Sojaöl, hydriertem Rapsöl, hydriertem Baumwollöl, hydriertem Palmöl, hydriertem Leinöl, Natriumglycerylmono-, -di-, - tristearat, Calciumstearat, Magnesiumstearat, Zinkstearat, Stearylalkohol und Cetylstearylalkohol ausgewählte hydrophobe Substanzen umfasst.

8. Mikrogranulat nach einem der vorhergehenden Ansprüche, wobei die mindestens eine erste Kernüberzugsschicht mindestens eine aus der aus pflanzlichem Lecithin, ethoxylierten Ölen oder Alginaten bestehenden Gruppe emulgierender Substanzen und/oder aus der aus Fettsäuren bestehenden Gruppe und/oder aus der aus Methacrylatpolymeren bestehenden Gruppe ausgewählte verunreinigende Substanz umfasst.

9. Mikrogranulat nach Anspruch 8, wobei die emulgierende Substanz aus der aus Soja- oder Sonnenblumenlecithin, ethoxyliertem Rizinusöl, Alkali- und Magnesiumalginaten bestehenden Gruppe ausgewählt ist.

10. Mikrogranulat nach Anspruch 8, wobei die verunreinigende Fettsäure aus der aus Palmitinsäure, Ölsäure, Linolsäure, Linolensäure, Stearinsäure oder einer Kombination davon bestehenden Gruppe ausgewählt ist.

11. Verfahren zur Herstellung eines Mikrogranulats nach einem der vorhergehenden Ansprüche, welches die folgenden Schritte umfasst:
- das Extrudieren einer eine oder mehrere physiologisch aktive Substanzen, ausgewählt aus der aus Methionin, Cholin, Cholinchlorid, Lysin, Lysinhydrochlorid, Lysinsulfat, Vitamin C, Vitamin A, Vitamin E, Vitamin D3, Vitamin B1, Vitamin B2, Vitamin B6, Vitamin B12, Vitamin H, Vitamin PP und einer Milchsäure, Fumarsäure, Sorbinsäure, Ameisensäure, Citronensäure, Äpfelsäure, Essigsäure, Buttersäure und Propionsäure umfassenden Mischung von Säuren bestehenden Gruppe, Bindemittel, inerte Stoffe und Extrusionsadjuvantien sowie Sprengmittel umfassenden Mischung und
- gegebenenfalls das Sphäronisieren des Mikrogranulats,
- das Ausbilden einer oder mehrerer Überzugsschichten.

12. Vormischung für Tierfuttermittel, umfassend ein Mikrogranulat nach einem der Ansprüche 1 bis 10.

13. Futtermittel, umfassend die Vormischung nach Anspruch 12.

## Revendications

1. Microgranule comprenant :
i) un noyau comprenant :
a) une ou plusieurs substances physiologiquement actives choisies dans le groupe constitué de méthionine, choline, chlorure de choline, lysine, chlorhydrate de lysine, sulfate de lysine, vitamine C, vitamine A, vitamine E, vitamine D3, vitamine B1, vitamine B2, vitamine B6, vitamine B12, vitamine H, vitamine PP et d'un mélange d'acides comprenant l'acide lactique, fumarique, sorbique, formique, citrique, malique, acétique, butyrique et propionique ; et
b) une matrice comprenant au moins l'une parmi une substance ayant une action de liaison et une substance inerte, dans lequel :
la substance ayant une action de liaison est d'origine végétale ou synthétique et est un caoutchouc, une cellulose, un amide, une cire ou une graisse ; et
la substance inerte est un silicate, une silice colloïdale, une silice amorphe synthétique, une silice précipitée, un silicate d'alumine sodique, un silicate de calcium, du talc, du kaolin, ou une zéolite synthétique hydrophobe ; et
c) au moins un agent désintégrant qui est une lécithine végétale choisie parmi la lécithine de soja et la lécithine de tournesol ;
dans lequel le au moins un agent désintégrant est présent en une quantité en poids comprise entre 1,5 % et 6,5 % par rapport au poids total du noyau
ii) au moins une première couche de revêtement comprenant une ou plusieurs substances hydrophobes choisies dans le groupe constitué de : graisses, acides gras, huiles hydrogénées, monoglycérides et diglycérides d'acides gras, sels d'acides gras (tels que le stéarate de zinc, de magnésium ou de calcium), esters d'acides gras et d'alcools gras et leurs mélanges.

2. Microgranule selon la revendication 1, dans lequel la quantité totale de la substance ayant une action de liaison et de la substance inerte est comprise entre 10 % et 30 % en poids du poids total du noyau.

3. Microgranule selon l'une quelconque des revendications précédentes, dans lequel l'au moins un agent désintégrant est la lécithine de soja.

4. Microgranule selon la revendication 1 ou la revendication 2, dans lequel l'au moins un agent désintégrant est la lécithine de tournesol.

5. Microgranule selon l'une quelconque des revendications précédentes, dans lequel ledit noyau a une forme cylindrique dont la hauteur est comprise entre 0,5 mm et 2 mm ou une forme sphéroïdale dont le diamètre est compris entre 0,5 mm et 2 mm.

6. Microgranule selon l'une quelconque des revendications précédentes, dans lequel le poids total des couches de revêtement est compris entre 15 % et 40 % du poids du microgranule.

7. Microgranule selon l'une quelconque des revendications précédentes, dans lequel l'au moins une première couche de revêtement de noyau comprend une ou plusieurs substances hydrophobes choisies parmi : acide laurique, acide stéarique, acide palmitique, stéarine, huile de soja hydrogénée, huile de colza hydrogénée, huile de coton hydrogénée, huile de palme hydrogénée, huile de lin hydrogénée, mono, di, tristéarate de glycéryle de sodium, stéarate de calcium, stéarate de magnésium, stéarate de zinc, alcool stéarylique et alcool cétylstéarylique.

8. Microgranule selon l'une quelconque des revendications précédentes, dans lequel la au moins une première couche de revêtement de noyau comprend au moins une substance polluante choisie dans le groupe des substances émulsifiantes constitué de la lécithine végétale, des huiles éthoxylées ou des alginates et/ou dans le groupe constitué des acides gras et/ou dans le groupe formé par les polymères de méthacrylate.

9. Microgranule selon la revendication 8, dans lequel ladite substance émulsifiante est choisie dans le groupe constitué de la lécithine de soja ou de tournesol, de l'huile de ricin éthoxylée, des alginates de métal alcalin et de magnésium.

10. Microgranule selon la revendication 8, dans lequel ledit acide gras polluant est choisi dans le groupe constitué de l'acide palmitique, l'acide oléique, l'acide linoléique, l'acide linolénique, l'acide stéarique ou une combinaison de ceux-ci.

11. Procédé de préparation d'un microgranule selon l'une quelconque des revendications précédentes, comprenant les étapes de :
- extrusion d'un mélange comprenant une ou plusieurs substances physiologiquement actives choisies dans le groupe constitué de méthionine, choline, chlorure de choline, lysine, chlorhydrate de lysine, sulfate de lysine, vitamine C, vitamine A, vitamine E, vitamine D3, vitamine B1, vitamine B2, vitamine B6, vitamine B12, vitamine H, vitamine PP et un mélange d'acides comprenant l'acide lactique, fumarique, sorbique, formique, citrique, malique, acétique, butyrique et propionique ; substances de liaison, substances inertes et adjuvants d'extrusion, et agents désintégrants ;
- éventuellement soumission du microgranule à une sphéronisation ;
- formation d'une ou plusieurs couches de revêtement.

12. Prémélange pour aliments pour animaux comprenant un microgranule selon l'une quelconque des revendications 1 à 10.

13. Aliment pour animaux comprenant le prémélange selon la revendication 12.
